# EUROPÄISCHE PATENTANMELDUNG

(11) **EP 1 262 587 A1**
(43) Veröffentlichungstag der Anmeldung: **04.12.2002**
(21) Anmeldenummer: 02011408.8
(22) Anmeldetag: 24.05.2002
(51) Int. Cl.: D04H 13/00, B32B 5/26, B32B 5/04, B32B 3/28, A61F 13/15

(54) **Schichtstoff und Verfahren zu seiner Herstellung**

(30) Priorität: 30.05.2001 DE 10126143
(71) Anmelder: Carl Freudenberg KG, 69469 Weinheim (DE)
(72) Erfinder: Groitzsch, Dieter, Dr., 69493 Hirschberg (DE); Schaut, Gerhard, Dr., 69502 Hemsbach (DE)

(57) **Zusammenfassung**

Beansprucht ist ein Schichtstoff, aufweisend eine erste Deckschicht, ein Durchbrechungen aufweisendes Flächengebilde als Mittelschicht und eine zweite Deckschicht sowie ein Verfahren zu seiner Herstellung und die Verwendung des Schichtstoffes als Fluidaufnahme- und Verteilerschicht aus einem in der Z-Richtung ausgerichteten Vliesstoffschichtstoff für absorbierende Hygiene-Artikel.

Eine dreidimensionale Form wird dadurch erreicht, daß das Flächengebilde in der Mittelschicht in geschrumpftem Zustand vorliegt bzw. in diesen gebracht wird.

## Beschreibung

### Technisches Gebiet

Die Erfindung betrifft einen Schichtstoff, aufweisend eine erste Deckschicht, ein Durchbrechungen aufweisendes Flächengebilde als Mittelschicht und eine zweite Deckschicht sowie ein Verfahren zu seiner Herstellung und die Verwendung des Schichtstoffes als Fluidaufnahme- und Verteilerschicht aus einem in der Z-Richtung ausgerichteten Vliesstoffschichtstoff für absorbierende Hygiene-Artikel.

### Stand der Technik

Der absorbierende Kern von Kinderwindeln, Inkontinenz- und Damenhygieneprodukten ist heute auf der Trageseite, d.h. der Körper zugewandten Seite von mindestens zwei Schichten abgedeckt. Zwischen dem Abdeckvliesstoff bzw. der perforierten Folie und dem absorbierenden Kern ist eine Aufnahme- und Verteilerschicht aus Vliesstoff oder reticuliertem Schaumstoff positioniert, die wie der Name schon ausdrückt die Körperflüssigkeit (Urin, dünner Stuhlgang oder Menses) schnell aufnimmt und möglichst gleichmäßig an den darunter liegenden, gewöhnlich aus Zellstoff und Superabsorber-Pulver bestehenden absorbierenden Kern verteilt. Dadurch wird einerseits die menschliche Haut trocken gehalten mit dem Ergebnis der Verhinderung von Hautreizungen und andererseits eine Leckage der Körperflüssigkeit durch seitlichen Austritt verhindert. Auf der Rückseite ist das absorbierende Hygiene-Produkt mit einer wasserdichten Folie oder einem Vliesstoff-Folienlaminat gegen Körperflüssigkeitsdurchtritt abgedichtet.

Für die Aquisitions- und Verteilerschicht sind thermisch in einem heißluftdurchströmten Trockner gebundene Vliesstoffe oder mit Polymerdispersionen gebundene Vliesstoffe aus gekräuselten, relativ grobtitrigen Fasern bekannt. Die Fasern weisen Titer von mehr als 3,3 dtex und bestehen vorzugsweise auf Polyester (Polyäthylenterephthalat) und/oder Polyolefinen, wobei zum Zwecke einer Faserbindung im Durchströmofen Bikomponenten-Fasern mit Side-By-Side- oder Kern/Mantelstruktur eingesetzt werden und eine der beiden Faserkomponenten deutlich tiefer schmilzt als die andere Komponente. Solche Vliesstoffe weisen in Beziehung zu ihrem niedrigen Gewicht unmittelbar nach deren Fertigung ein relativ hohes Volumen und insbesondere eine große Dicke auf. Es ist jedoch bekannt, daß diese anfängliche Dicke bereits beim Aufrollen der Ware unter praxisüblichen Spannungen deutlich herabgesetzt wird und die Preßbedingungen in der Verpackung ein weiteren Beitrag zur Dickenverminderung leisten.

Es wurden deswegen Lösungen angestrebt, eine Dicke nicht nur allein durch mehr oder weniger statistisch verteilte gekräuselte Fasern und deren Bindung zu erreichen, sondern solche gekräuselten Faservliesstoffe durch Wellung oder andere geometrische Ausrichtungen in die dritte Dimension, die mit Z-Richtung beschrieben wird, zu bringen. Es hat sich gezeigt, daß dadurch höhere Kompressionswiderstände erreicht werden können als mit sog. High-Loft-Vliesstoffen mit der Folge eines deutlich geringeren Dickenverlustes beim Durchschreiten der Fertigungsstufen einer Windel, einschließlich Verpacken und Lagern.

In der WO 92/01401 (US 5.611.791) wird ein Schichtstoff aus Folie und plissierten Faserflorlagen beschrieben. Der Schichtstoff besteht aus einer Polypropylen-Monofilamentfolie und gewellten oder in anderen geometrischen Strukturen in die Z-Richtung ausgerichteten Polypropylen-Fasern. In den Tälern der Wellen sind die Polypropylen-Fasern mit den Polypropylen-Monofilamenten innig miteinander verschweißt.

Das Verfahren zur Herstellung eines solchen Monofilament Vliesstoff - Schichtstoffes ist in WO 98/06290 beschrieben. Die kardierten Polypropylen-Stapelfaserflore passieren den Spalt zwischen zwei heißen, ineinandergreifenden Positiv- und Negativwalzen, werden dort in Wellenform gebracht und nach dem Verlassen des Kalanderwalzenspaltes durch Aufblasen und Absaugen heißer Luft in dieser Form gehalten, bis die Wellen schließlich durch Anpressen der zugeführten Polypropylenfolie zwischen einer glatten und einer gewellten Walze an den erhaben Stellen derselben zu Verschweißungen zwischen Polyproyplen-Fasern der Folie kommt. Nachteil dieses Verfahrens und der daraus resultierenden gewellten Schichtstoffes ist die Tatsache, daß die Wellenhöhe im Produkt ausschließlich durch die Wellenhöhe der ineinandergreifenden Positiv- und Negativ-Kalanderwalzen bestimmt wird und diese starken Limitierungen unterliegt.

Ein solcher Schichtstoff weist zwar die gewünschte Wellung auf, ist aber als Aquisitions- und Verteilerschicht ungeeignet, weil die Folie eine Weitergabe der Körperflüssigkeit an den absorbierenden Kern verhindert.

### Darstellung der Erfindung

Mit der Erfindung wurde die Aufgabe gelöst die oben genannten Nachteile sowohl bezüglich des Verfahrens als auch bezüglich des Schichtstoffs zu beseitigen und dem Schichtstoff verbesserte Eigenschaften für das Körperflüssigkeitsmanagement zu verleihen.

Gemäß der Erfindung wird ein Schichtstoff vorgeschlagen, aufweisend eine erste Deckschicht, ein Durchbrechungen aufweisendes Flächengebilde als Mittelschicht und eine zweite Deckschicht, wobei das Flächengebilde in geschrumpften Zustand vorliegt.

Unter Flächengebilde ist ein zur Schrumpfung fähiges Plastiknetz oder Gelege oder anderes schrumpffähiges, mit Durchbrechungen versehenes flächiges Material zu verstehen. Zur Vereinfachung wird künftig immer von Netz gesprochen, selbst wenn damit auch schrumpffähige Gelege oder andere Flächengebilde mit Durchbrechungen gemeint sind. Unter Gelege werden hier solche Netze, deren Fäden sich an den sich kreuzenden Stellen mit Hilfe eines zusätzlichen Haft- oder Bindemittels aneinander gebunden sind verstanden. Die sich kreuzenden Fäden bzw. Mono- oder Multifilamente können aus gleichem oder unterschiedlichen Polymermaterial und von gleicher oder unterschiedlicher Stärke sein. Unter Plastiknetz ist dagegen hier ein Netz definiert, deren sich kreuzende Fäden durch die eigene Polymermasse miteinander verschweißt oder aneinander gebunden sind.

In einer besonderes Ausgestaltung der Erfindung werden solche Flächengebilde eingesetzt, die in Maschinenlaufrichtung eine sehr hohe und in Querrichtung eine sehr niedrige bzw. überhaupt keine Schrumpfkraft besitzen. Bekannt sind u.a. solche Netze, die entweder aus demselben Thermoplasten bestehen, deren Fäden in Maschinenlaufrichtung jedoch um ein Vielfaches dicker bzw. schwerer sind als in Querrichtung oder solche Netze, die aus zwei unterschiedlichen Polymeren bestehen, wobei deren Fäden in Maschinenlaufrichtung aus schrumpffähigen und die in Querrichtung aus unter Prozeßbedingungen nicht schrumpffähigem Material aufgebaut sind.

Dies kann auch mit gleichen Polymeren dadurch geschehen, dass das Netz nur in Maschinenlaufrichtung bzw. in Maschinenlaufrichtung mehr als in Querrichtung verstreckt wird. Ein höherer Verstreckungsgrad führt zu einem höheren Schrumpf.

In einer besonderen Ausführungsform werden Netze mit sehr groben Monofilamenten in Längs- und sehr feinen Monofilamenten in Querrichtung aus demselben Polymer, wie beispielsweise Polypropylen eingesetzt. Die Prozeßbedingungen der Kalandrierung werden so gewählt, daß die sehr feinen Querfäden des Netzes aufschmelzen und sich dessen Schmelzmasse an den Längsfäden ansammelt. In dieser speziellen Ausführungsform entsteht ein Schichtstoff, mit parallelen ausgeprägten Querwellen, beidseitig des geschrumpften Netzes.

Dadurch, daß die erste und die zweite Deckschicht aus Fasern und/oder Filamenten bestehen, deren Schmelz- oder Erweichungspunkt mindestens 30°C über dem Schmelz- oder Erweichungspunkt des Flächengebildes liegt, ist nur das Flächengebilde für die Schrumpfung verantwortlich.

Vorteilhafterweise besteht mindestens eine Deckschicht aus gekräuselten Stapelflaserfloren oder Endlosfilamenten. Es ist ebenso möglich, daß mindestens eine Deckschicht aus einem vorgebundenen Flächengebilde, beispielsweise Papier oder Vliesstoff, besteht. Dadurch, daß in mindestens einer Deckschicht zumindest anteilig thermoplastisch verformbares und verschweißbares Material vorhanden ist, ist ein Verschweißen des Schichtstoffes zum Zweck der Verbindung der einzelnen Schichten untereinander möglich.

Eine der beiden gekräuselten Stapelfaserflore oder Endlosfilamentschicht kann durch ein anderes bereits vorgebundes Flächengebilde, wie Papier oder Vliesstoff ersetzt werden. Auch in diesem Fall gilt, daß keine Faser- oder sonstige Komponente mit einem Schmelz- oder Erweichungspunkt unterhalb dem des Netzes verwendet wird. Mindestens eine der beiden das Netz umhüllende Schichten muß zumindest anteilig thermoplastisch verformbare und verschweißbare Fasern oder sonstigen Komponenten enthalten. Eine oder beide der Schichten kann zumindest anteilig nicht schmelzende, dauerhaft fluidsaugende Fasern, wie z.B. Zellwolle; Baumwolle, Wolle, Lyocell und dergleichen enthalten. Enthält eine der beiden Schichten keinerlei unter Prozeßbedingungen durch Hitze und Druck verschweißbare Fasern, so muß zumindest die andere Schicht eine solche Faser enthalten.

Das Flächengebilde in der Mittelschicht kann ein Gelege, dessen Fäden sich an den sich kreuzenden Stellen mit Hilfe eines zusätzlichen Haft- oder Bindemittels aneinander gebunden sind, oder ein Netz aus Polymermasse, deren sich kreuzende Fäden durch die eigene Polymermasse miteinander verschweißt oder aneinander gebunden sind, sein.

Die sich kreuzenden geradlinigen Fäden des Flächengebildes können in einem Winkel von 90° zueinander angeordnet sein.

Vorteilhafterweise ist das Flächengebilde in einer Vorzugsrichtung aus schrumpffähigen und in einer bezogen auf die Vorzugsrichtung verlaufenden Querrichtung aus unter Prozeß- und Anwendungsbedingungen nicht schrumpffähigen Material gebildet.

Das Flächengebilde kann aus sehr groben verstreckten Monofilamenten in Längs- und sehr feinen Monofilamenten in Querrichtung aus demselben Polymer, beispielsweise Polypropylen, gebildet sein. Im Schichtstoff kann sich in diesem Fall an den Längsfäden die Schmelzmasse der aufgeschmolzenen Querfäden ansammeln.

Ein weiterer Gegenstand der Erfindung ist ein Schichtstoff, aufweisend eine erste Deckschicht, eine Mittelschicht aus sich nicht überkreuzenden Fäden und eine zweite Deckschicht, wobei die sich nicht überkreuzenden Fäden in geschrumpften Zustand vorliegen.

Vorteilhafterweise umfasst der Schichtstoff drei Schichten, wobei die mittlere Schicht aus längs ausgerichteten parallelen Monofilamentfäden aus einem ersten Thermoplasten besteht und wobei ober- und/oder unterhalb der Fäden des ersten Thermoplasten Monofilamentfäden aus einem zweiten Thermoplasten abgelegt sind, wobei die zweiten Monofilamentfäden derart angeordnet sind, daß mindestens zwei parallel und linear ausgerichteten Fäden des ersten Thermoplast gekreuzt und verbunden sind.

Anstelle eines Netzes können auch schrumpffähige Längsfäden (Strands) oder generell sich nicht kreuzende Fäden jeder Form als mittlere Schicht eingetragen werden. Die Monofilamentfäden können von einem Kettbaum zugeführt werden. Vorzugsweise werden sie jedoch direkt durch Extrusion nach bekannten Verfahren auf die untere Kompositschicht in paralleler Anordnung aufgetragen, bevor dieselbe mit einer weiteren Schicht oben abgedeckt wird. Es ist auch denkbar durch Schwenkung der aus der Düse austretenden Monofilamente eine bogenförmige Ablage der Monofilamente zu erreichen. Zur Erzeugung einer gut ausgeprägten Wellenstruktur der beiden durch Schrumpf der Monofilamentfäden aufgerichteten Außenschichten ist es jedoch vorteilhaft, wenn sich die Bögen der Monofilamente nicht kreuzen. Dies kann durch den Abstand der Düsenbohrungen und der Bogenamplitude gesteuert werden.

Dabei sollten die ersten Monofilamentfäden einen um mindestens 30° höheren Schmelz- oder Erweichungspunkt aufweisen.

Vorteilhafterweise sind die zweiten Monofilamentfäden sinusförmig über die längs ausgerichteten ersten Monofilamentfäden abgelegt.

Dabei können die zweiten Monofilamenten Bikomponenten-Struktur aufweisen, wobei zumindest eine Polymerkomponente eine um mindesten 30° gegenüber den ersten Monofilamentfäden aus einem ersten Thermoplast höheren Schmelz- bzw. Erweichungspunkt aufweist.

Vorteilhafterweise beträgt der Schrumpf in Längsrichtung 20 bis 75 %, vorzugsweise 30 bis 60 % bezogen auf die Länge der ungeschrumpften Ausgangsbasis.

Der Schichtstoff kann ein Flächengewicht von 50 bis 200 g/m², vorzugsweise 70 bis 150 g/m² aufweisen, wobei die zweite Schicht 5 bis 50 %, vorzugsweise 10 bis 30 % des Gesamtgewichts ausmachen.

Vorteilhafterweise liegt der Schmelz- oder Erweichungspunkt der Fasern der Deckschicht mindestens 20 bis 30°C über dem der Mittelschicht bzw. dem niedrigst schmelzenden Teil der Mittelschicht und weist keinen oder im Vergleich zur Mittelschicht nur einen unbedeutenden Flächenschrumpf auf.

Vorteilhafterweise sind die erste und/oder die zweite Schicht aus hydrophilen Faserfloren gebildet, wobei vorzugsweise ein Hydrophilie-Gradient zwischen der ersten und der zweiten Deckschicht vorhanden ist.

Ein weiterer Gegenstand der Erfindung ist die Verwendung des Schichtstoffs für eine Fluidaufnahme- und/oder Verteilerschicht für absorbierende Hygiene-Artikel.

Schließlich ist ein Verfahren zur Herstellung eines mindestens drei Schichten aufweisenden Schichtstoffs Gegenstand der Erfindung, bei dem zwischen zwei Deckschichten eine Mittelschicht in Form eines Flächengebildes oder in Form von Fäden eingebracht wird. Gemäß der Erfindung wird in einem zweiten Schritt das Flächengebilde oder die Längsfäden bei einer Temperatur oberhalb der Schmelz- oder Erweichungstemperatur des Materials der Mittelschicht im Walzenspalt geschrumpft.

Zur Verbindung der einzelnen Schichten ist mindestens eine Walze mit einem unterbrochenen Muster, beispielsweise Punkten, graviert.

Vorteilhafterweise werden die drei Schichten einem Kalander mit mindestens einer beheizten, gravierten Stahlwalze zugeführt und dort zu einem Schichtstoff verbunden, wobei die Verbindung insbesondere als Vernieten zu bezeichnen ist.

In dem Kalanderspalt können die Querfäden durchgeschmolzen, durchgetrennt bzw. stark geschädigt werden.

Weiterhin können die Fäden bogenförmig auf der ersten Deckschicht abgelegt werden, wobei die Fäden vorzugsweise untereinander kreuzungsfrei abgelegt werden.

### Kurzbeschreibung der Zeichnungen

In den Zeichnungen ist ein erfindungsgemäßer Schichtstoff und das erfindungsgemäße Verfahren verdeutlicht. Es zeigt die:
- Fig. 1: das Verfahren für die Herstellung eines geschrumpften Schichtstoffes, die
- Fig. 2: einen Schichtstoff gemäß der Erfindung mit einem ausschließlich in Längsrichtung geschrumpften Flächengebilde, die
- Fig. 3: einen Ausschnitt aus Fig. 2 in Vergrößerung, die
- Fig. 4: den Längsschnitt von Fig. 2 entlang der Linie A-A, die
- Fig. 5: den Längsschnitt von Fig. 2 entlang der Linie B-B, die
- Fig. 6: Querschnitt von Fig. 2 entlang der Linie C-C, die
- Fig. 7: einen Schichtstoff mit komprimierten Stapelfaser- oder Spinnvliesstoff mit einem annähernd rechteckigen Querschnitt, die
- Fig. 8: einen Vergleich zu einem nicht gebündelten Vliesstoff und
- Fig. 9: den Eintrag der komprimierten Vliesstoffstreifen aus Fig. 7 in den Schichtstoff.

### Ausführung der Erfindung

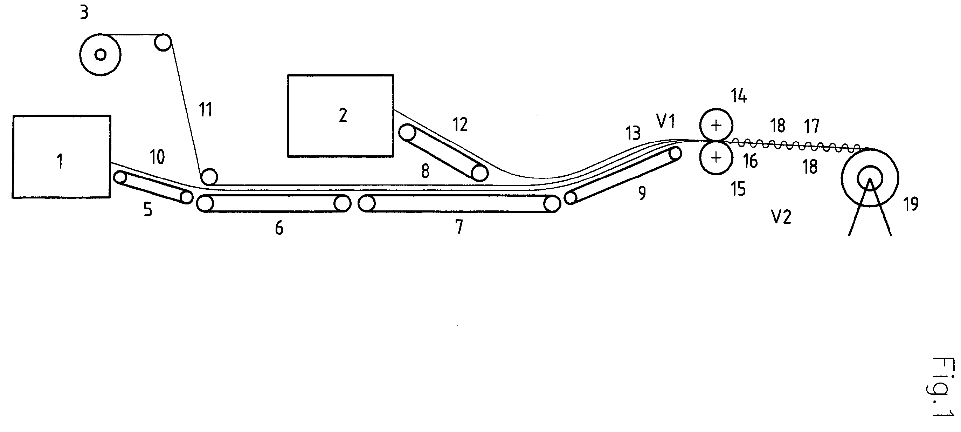

In Fig.1 ist das Verfahren für die Herstellung eines geschrumpften Schichtstoffes, bestehend aus einem ersten Stapelfaserflor, einem Netz und einem zweiten Stapelfaserflor schematisch dargestellt.

Aus einer Krempelmaschine 1 wird auf das Förderband 5 ein loser erster Faserflor 10 aus gekräuselten Fasern entweder in Maschinenrichtung oder quer zur Maschinenrichtung oder in einer Wirrlage aufgelegt und zu den sich an das Förderband 5 anschließenden Förderbändern 6, 7 und 9 weiter transportiert. Auf diesen losen ersten Faserflor 10 wird ein von einer Rolle 3 über Umlenkwalzen geführtes Flächengebilde 11 abgelegt und bildet die Mittelschicht. Das Flächengebilde weist Durchbrechungen auf und kann als Netz oder Gelege ausgeführt sein. Dieses Flächengebilde 11 wird schließlich mit einem zweiten Faserflor 12 von einer Krempelmaschine 2 über das Förderband 8 kommend abgedeckt.

Dieser 3-lagige, völlig ungebundene und durch die Förderbänder 7 und 9 gestützte Verbund 13 passiert schließlich einen Walzenspalt 16 (Nip oder Kalandernip), der durch zwei heiße Kalanderwalzen 14 und 15 begrenzt ist. Die Kalanderwalze 15 ist eine gravierte Walze mit unterbrochenen Muster (wie beispielsweise Punkte) und die Kalanderwalze 14 ist eine glatte Stahlwalze.

Die Temperaturen der beiden Walzen 14 und 15 werden im Normalfall auf annähernd gleiches Niveau eingestellt, wenn beide Flore 10, 12 aus Fasern des gleichen Schmelz- bzw. Erweichungspunktes bestehen. In einem anderen Fall können die Temperaturen variieren. Zumindest eine der beiden Walzentemperaturen muß jedoch mindestens 20-30°C über der Schmelztemperatur des Flächengebildes 11 liegen. Beim Verlassen des Walzenspalts 16 entsteht der punktverschweißte und gleichzeitig in Maschinenrichtung geschrumpfte Schichtstoff 17 mit wellenförmigen Erhebungen 18, die sich abwechselnd beidseitig der Mittelebene des Flächengebildes 11 erstrecken.

Zwischen dem Beginn oder Ende des Förderbandes 9 und dem Austritt aus dem Kalandernip 16 kann zusätzlich ein Breitstreckhalter, z.B. ein Spannrahmen, installiert sein, um Schrumpf in der Querrichtung zu verhindern. Dieser Breitstreckhalter ist in Fig. 1 nicht eingetragen.

Die das Netz umhüllenden Faserflore 10, 12 bestehen zu 100% aus solchen Fasern, deren Schmelz- oder Erweichungspunkt deutlich, d.h. mindestens ca.30°C, über der Komponente des Flächengebildes 11 liegt, welches für die thermische Schrumpfung verantwortlich ist.

Die Aufwickelgeschwindigkeit V₂ an einem nach den Kalanderwalzen 14, 15 angeordneten Aufroller 19 verringert sich um den Längschrumpfbetrag des Flächengebildes gegenüber V₁ vor dem Kalander. Beträgt V₁ beispielsweise 100 m/min. und der Längsschrumpf 50%, so reduziert sich die Aufrollgeschwindigkeit V₂ auf 50 m/min. Es kann angebracht sein, zwischen den Kalanderwalzen 14 und 15 und dem Aufroller 19 einen Warenspeicher zu installieren, um eine möglichst spannungsarme Aufrollung zu gewährleisten.

Die Krempelmaschine 1 zur Ablage des Stapelfaserflors 10 kann ersetzt sein durch einen Extruder, eine Spinndüsenanordnung und eine gerichtete Luftstromführung zum Zwecke der Filamentabschreckung und Verstreckung zu einem Endlosfilamentvliesstoff (Spinnvliesstoff) nach bekannten Verfahren, wobei die Düsenbohrungen das Erspinnen von Monofilamenten oder vorzugsweise Bikomponentenfaser mit Kern/Mantel- oder ganz besonders bevorzugt, mit Side-By-Side-Struktur, welche bekanntermaßen schon beim Abschreckvorgang eine gewünschte Spiralkräuselung auslösen, gestatten. Die Düsenbohrungen und Luftverstreckung werden so gewählt, daß relative grobtitrige Filamente von über ca. 10 dtex entstehen.

In einer weiteren Ausgestaltungsform des Verfahrens kann der lose Faserflor 10 aus Stapelfasern oder wirr abgelegten Endlosfilament durch einen bereits vorgebunden Vliesstoff ersetzt werden. Für das Produkt sind auch in diesem Fall grobe Fasern vorteilhaft.

Im Walzenspalt 16 der gravierten Kalanderwalze 14 und glatten Kalanderwalze 15 werden die beiden Vliesstofflagen 10 und 12 punkt- oder unterbrochen musterartig verschweißt. Die Wahrscheinlichkeit, daß Kalanderschweißpunkte auch das Flächengebilde 11 mit einschließen sind äußerst gering.

Das Flächengebilde kann durch in Längsrichtung ausgerichtete Monofilamentfäden ersetzt werden, die parallel zueinander ausgerichtet und in einem gleichbleibenden Abstand aufgelegt werden können. Die Fäden können auf einem Kettbaum aufgewickelt sein und werden von dort in immer gleichmäßigem Abstand zueinander auf den unteren Faserflor 10 abgelegt. Der lose untere Faserflor 10 kann auch durch eine bereits vorverfestigten Vliesstoff ersetzt sein. Die Monofilamente können aber auch direkt aus der Polymerschmelze auf die untere Vliesstofflage aufextrudiert werden.

Es ist auch denkbar, zusätzlich zu den längs ausgerichteten parallelen Monofilamentfäden aus einem ersten Thermoplasten und aus einem zweiten Extruder und einer zweiten Düsenplatte Monofilamentfäden aus einem zweiten Thermoplasten ober- und unterhalb der Fäden des ersten Thermoplasten abzulegen, wobei der Düsenbalken aus dem die Monofilamente des zweiten Thermoplasten austreten einer oszillierenden Seitwärtsbewegung unterzogen ist. Die Schwingungsamplitude der seitlichen Oszillation ist so gewählt, daß am Auftrittsort jeweils ein sinusförmig abgelegter Faden aus dem zweiten Thermoplasten mindestens zwei parallel und linear ausgerichteten Fäden des ersten Thermoplasten kreuzt und verbindet.

Die ersten Thermoplast-Monofilamentfäden weisen einen um mindestens 30°C höheren Schmelz- oder Erweichungspunkt auf. Der erste Thermoplast kann beispielsweise aus Polypropylen mit einem Schmelzpunkt von ca. 165°C und der zweite aus Polyäthylenterephthalat mit einem Schmelzpunkt von ca. 260°C bestehen. Die zweiten durch Oszillation sinusförmig abgelegten Filamente können auch Bikomponenten-Strukur, wie Side-By-Side- oder Kern/Mantel-Struktur aufweisen. In diesem Fall ist zumindest eine Polymerkomponente im Schmelz- bzw. Erweichungspunkt um mind. 30°C höher als der erste Thermoplast der parallel orientierten Monofilamentfäden.

Die Kalandertemperatur zur Verfestigung und gleichzeitigen Schrumpfung des Schichtstoffes wird so gewählt, daß ausschließlich die parallelen Filamentfäden in Längsrichtung aus dem ersten Thermoplasten am Austrittsspalt einem Schrumpf unterzogen werden. Die dadurch erreichte Wellung des Schichtstoffes ist eine besonders bevorzugte Ausgestaltung der Erhöhungen in Z-Richtung, die dem erfindungsgemäßen Produkt eine besonders hohe Stabilität gegen Druck- bzw. Druck- und Temperaturbelastung verleiht.

Ein solcher ausschließlicher Schrumpf in Längsrichtung kann auch mit einem Netz erreicht werden, wenn das erfindungsgemäße Verfahren durch ganz spezielle Gravurwahl der Kalanderwalze so modifiziert wird, daß im Kalanderspalt 16 die Querfäden durchgeschmolzen, durchgetrennt bzw. sehr stark geschädigt werden. Zu diesem Zwecke kann die Kalanderwalze beispielsweise unterbrochene Längsrillen mit extrem niedrigem oberstem Rillendurchmesser aufweisen, was die Schneid- bzw. Durchtrennwirkung erhöht. Diese unterbrochenen Längsrillen- bzw. Stäbchen können das alleinige Strukturelement der gravierten Walze sein. Diese geometrische Anordnung der Gravur zum hauptsächlichen Zwecke der Durchtrennung der Querfäden kann auch mit solchen Gravuren kombiniert sein, die hauptsächlich zur Laminatverbundfestigkeit beitragen. Beim Einsatz solcher speziellen, Querfäden durchtrennender Walzen werden längsausgerichtete Faserflore bevorzugt, wobei mindestens einer der beiden ein Längsflor sein sollte.

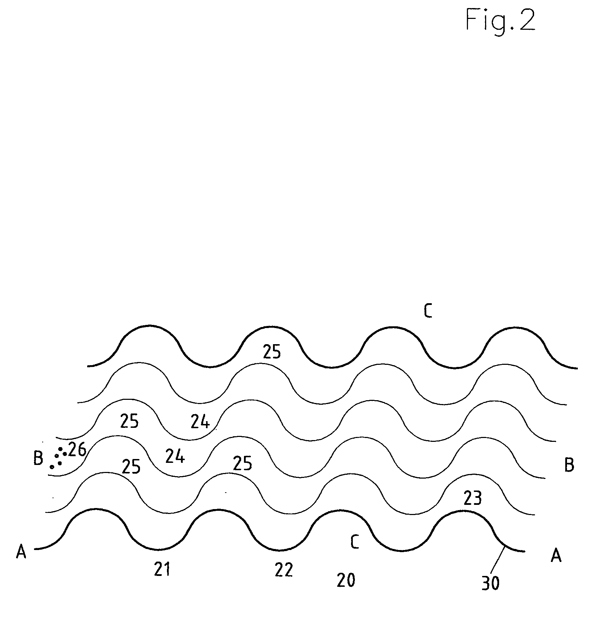

In Fig. 2 ist ein Schichtstoff 20 gemäß der Erfindung mit einem ausschließlich in Längsrichtung geschrumpften Flächengebilde dargestellt. Die Längsrichtung des Flächengebildes erstreckt sich von A nach A bzw. B nach B, die Querrichtung von C nach C. Die Oberfläche des geschrumpften Schichtstoffes ist in Längsrichtung wellenförmig und umfaßt Wellentäler 21, 22 und Wellenspitzen 23. Der Schichtstoff 20 ist in einem Bereich 25 mit den parallel ausgerichteten Monofilamentfäden 30 verstärkt. Zwischen den Monofilamentfäden 30 ist ein Bereich 24 vorhanden, in welchem Schweißpunkte 26 angeordnet sind.

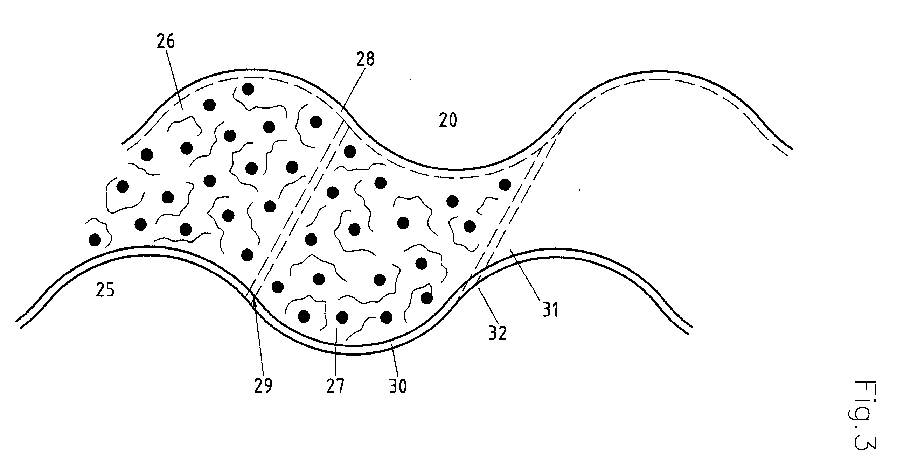

In Fig. 3 ist ein Ausschnitt aus Fig. 2 in Vergrößerung wiedergegeben. In den Flächen bzw. Räumen 24 zwischen den Monofilamentfäden 30 befindet sich eine Ober- und eine Unterschicht 31, 32 aus Fasern 27, die durch Schweißpunkte 26 miteinander verbunden sind. Jeweils am Wendepunkt der sinusförmigen Wellungen ist zwischen den beiden Vliesstoffschichten 31, 32 jeweils ein Querfaden 28 eingebettet, der an der Stelle 29 den Längsfaden 30 des Flächengebildes kreuzt und mit demselben verschweißt ist. In den Zonen zwischen den Verschweißpunkten 26 sind die Fasern 27 gewöhnlich nicht gebunden. Sie können jedoch in einer besonderen Ausführungsform auch durch Bindemittel kohäsiv gebunden sein.

Der geschrumpfte Längsfaden 30 befindet sich in der Mitte des Schichtstoffes 20. Die Querfäden 28 sind ungeschrumpft, so daß der Schichtstoff 20 vor und hinter dem Kalanderspalt dieselbe Breite aufweist.

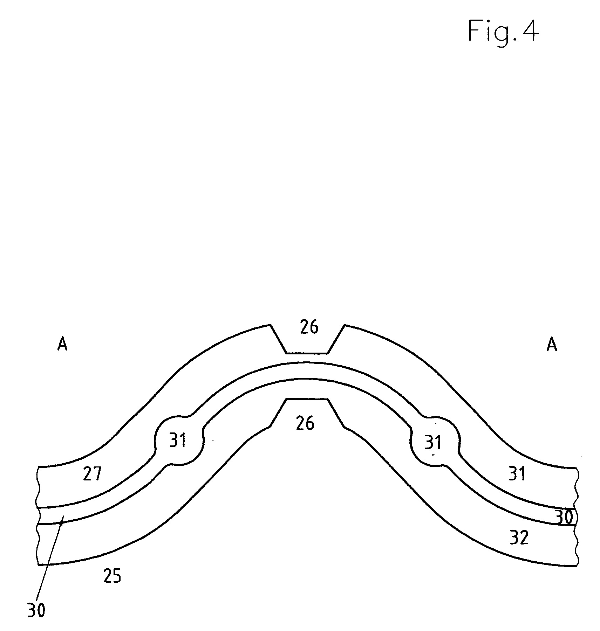

In Fig. 4 ist der Längsschnitt von Fig. 2 entlang der Linie A-A wiedergegeben. In der Mitte des Schichtstoffes befindet sich der geschrumpfte Längsfaden 30 eines Plastiknetzes. An der Kreuzungsstelle 29 (siehe Fig. 3) der miteinander verschweißten Längs- und Querfäden 30, 28 des Netzes ist eine Verdickung 31 dargestellt. Die Fasern 27 der beiden Vliesstofflagen sind an den Verschweißstellen 26 verdichtet und aneinander gebunden. Da die Verschweißstellen 26 in einer anderen räumlichen Ebene angeordnet sind als der dargestellte Längsschnitt, sind die Randkonturen der Verschweißstellen gestrichelt gezeichnet.

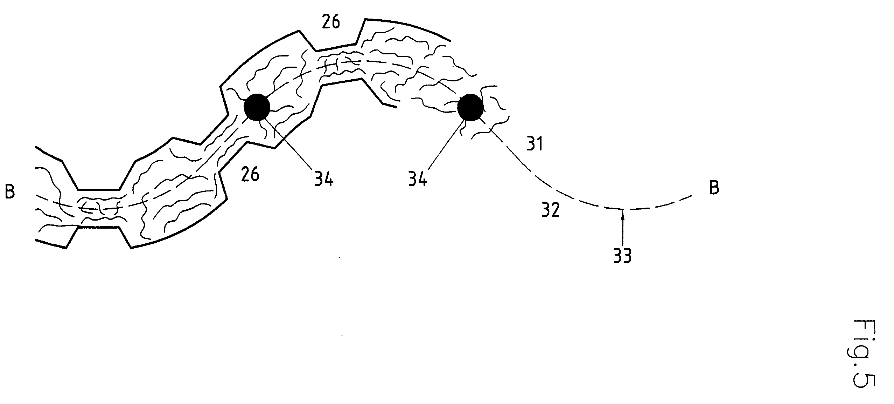

In Fig. 5 ist der Längsschnit von Fig. 2 entlang der Linie B-B wiedergegeben, d.h. in einem Bereich zwischen den Längsfäden 30 des Netzes. Die Querfäden 28 des Plastiknetzes sind hier als Querschnitt 34 dargestellt, der Verlauf der Längsfäden wird mit 33 bezeichnet.

Die Faserschichten 31 und 32 können qualitativ und quantitativ gleich oder ungleich sein. Sie können aus thermoplastischen Synthesefasern oder Verschnitten aus solchen mit nicht schmelzbaren hydrophilen Fasern wie Zellwolle, Lyocell, Baumwolle, Flachs etc. und/oder Wolle bestehen.

Kalanderverfestigbare Thermoplastfasern müssen zu einem Anteil von mindestens 20% zumindest in einer der beiden Vliesstoffschichten 31 oder 32 enthalten sein, um eine ausreichende Verbundhaftung nach der Kalandrierung zu gewährleisten.

Für das Polymermaterial des Längsfadens 30, der gegebenenfalls im Netz angeordnet ist, gibt es nur die eine Beschränkung, daß es einen Schmelz- oder Erweichungspunkt von mindestens 30°C unter dem der Thermoplastfaser der Schichten 31 und/oder 32 aufweist, welche die Verbundhaftung der beiden Schichten durch die Prägekalandrierung erbringt.

Die Längsfäden an sich oder im Netz bzw. Gitter können beispielsweise aus Polyolefinen, wie Polyäthylen und Polypropylen, Copolymerisaten aus Äthylen und Propylen, aus Polyamid 6, aus Copolyamiden, Copolyestern etc. oder auch aus thermoplastischen Elastomeren bestehen. Ihr Abstand zum nächst benachbarten Längsfaden beträgt im geschrumpften und ungeschrumpften mindestens 2 mm und höchstens 15 mm. Der Titer der Längsfäden bewegt sich im Bereich von 80 bis 2.000 dtex, vorzugsweise ca. 100 bis 1.000 dtex.

Der Schrumpf in Längsrichtung beträgt 20 bis 75%, vorzugsweise 30 bis 60% bezogen auf die Länge der ungeschrumpften Ausgangsbasis.

Der Titer der gekräuselten, durch Temperatur und Druck zur Bindung fähiger Fasern 27 der Schichten 31 und 32 des Schichtstoffes bewegt sich zwischen 6,7 und 50 dtex, vorzugsweise zwischen 15 bis 25 dtex. Mindestens ein 50%iger Anteil bezogen auf die Summe der Fasergewichte der Schichten 31, 32 besteht aus gekräuselten Fasern mit hoher Sprungkraft, wie beispielsweise Polyäthylenterephthalat oder Polypropylen.

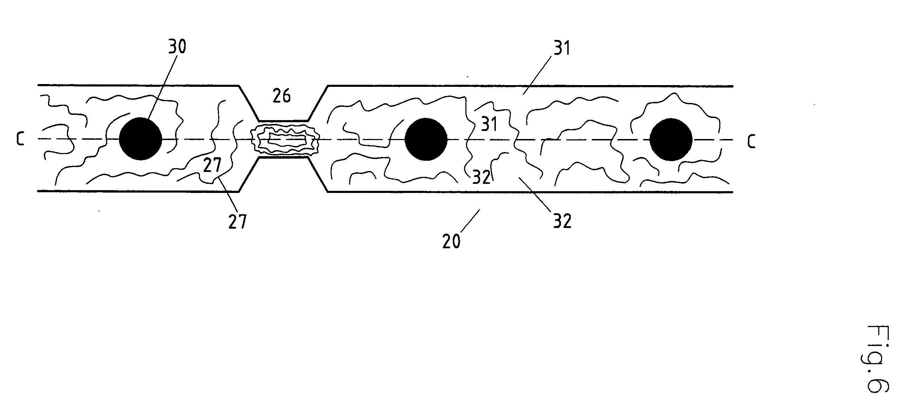

In Fig. 6 ist der Querschnitt des Schichtstoffes 20 entlang der Linie C-C dargestellt. Die Monofilamente 30 sind von den beiden Faserschichten 31 und 32 umschlossen. Die Fasern 27 der beiden Schichten 31 und 32 sind an den Verschweißstellen 26 stark verdichtet und aneinander gebunden.

Anstelle von Filamenten mit kreisrunden Querschnitt können auch solche mit jedem beliebigen Querschnitt verwendet werden, sie z.B. oval, rechteckig, rechteckig mit abgerundeten Ecken, dreieckig (trilobal) oder auch multilobal.

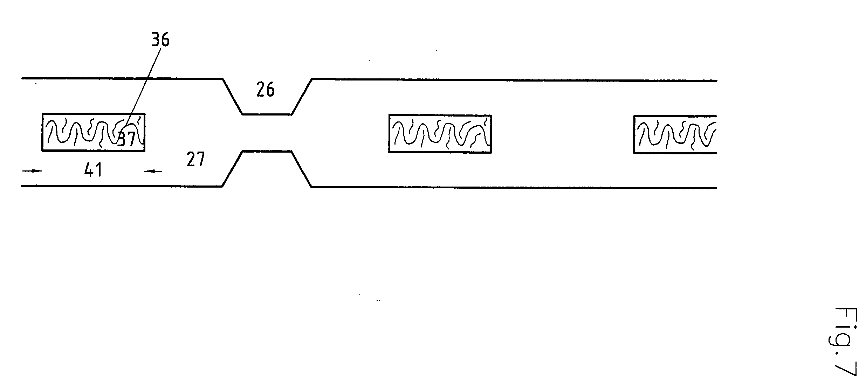

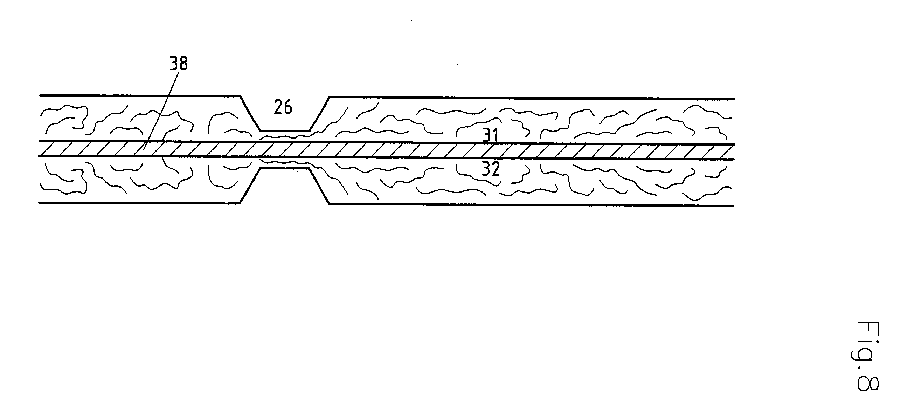

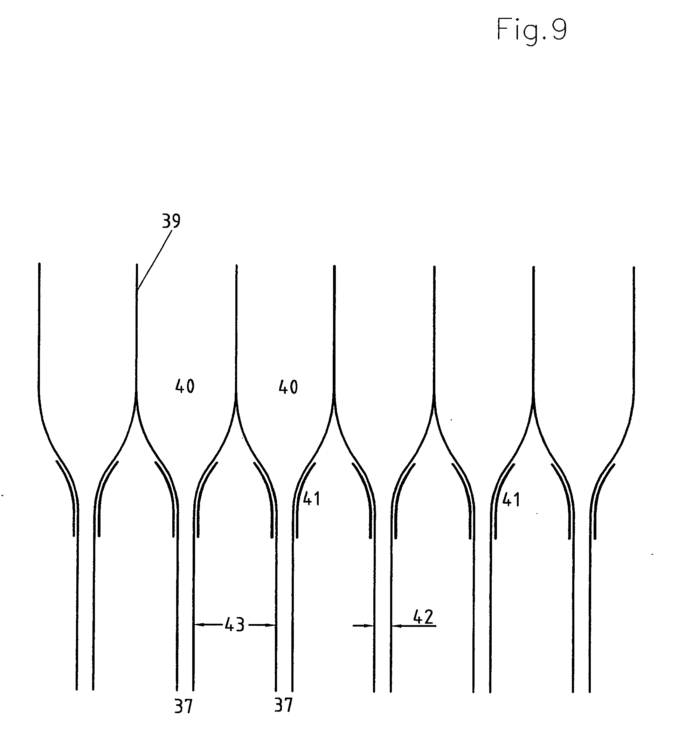

In Fig. 7 sind die Monofilamente 30 der Fig. 6 durch komprimierten Stapelfaser - oder Spinnvliesstoff 37 ersetzt worden mit einem annähernd rechteckigen Querschnitt. Wie diese komprimierten Vliesstoffstreifen 37 in den Schichtstoff 20 eingetragen werden, ist in Fig. 9 dargestellt. Ein leichter, ca. 6-10 g/m² schwerer, in Längsrichtung ausgekämmter Stapelfaserflor 38 wird an den Stellen 39 mit einem Schneidflorwerk in gleich breite schmale Stapelfaserflore 40 geschnitten. Diese werden anschließend, beispielsweise durch seitliche schwache Luftströme, zu einem sehr schmalen Flor 37 der Breite 42 zusammengeführt und durch eine auf einer Seite aufgeweitetes, auf Hochglanz poliertes Rohr 41 durchgeführt und schließlich in Längsrichtung auf dem unteren Trägerflor bzw. leicht vorverfestigem Vliesstoff in gleichmäßigen Abständen 43 abgelegt. Durch die Bündelung zu einem dicken Faserflor 37 wird die Schrumpfkraft gegenüber einem nicht gebündelten Vliesstoff und die Wellenhöhe gegenüber dem im Fig. 8 wiedergegebenen Fall deutlich erhöht. Hier wurde ein Längsflor 38 - d.h. ohne Bündelung - als schrumpfendes Flächengebilde zwischen die beiden Faserflore 31 und 32 gelegt.

Das Produkt hat ein Gewicht von 50 bis 200 g/m², vorzugsweise 70 bis 150g/m², wobei die als Schrumpfmedium eingesetzte Mittelschicht, unabhängig davon, ob nur Anteile davon schrumpfen, einen Anteil von 5 bis 50%, vorzugsweise 10 bis 30% bezogen auf das Gesamtgewicht des Schichtstoffes ausmachen.

Als Stapelfasern können allen bekannten synthetischen Monofilfasern eingesetzt werden, sofern sie einen Erweichungs- oder Schmelzpunkt aufweisen, der mindestens 20-30°C über dem der Mittelschicht bzw. dem niedrigst schmelzenden Anteil der Mittelschicht liegt und unter den Kalanderbedingungen selbst entweder keinen oder im Vergleich zur Mittelschicht nur einen unbedeutenden Flächenschrumpf ergeben. Auch synthetische Bi- oder Multikomponenten-Fasern sind einsetzbar, wobei in diesem Fall für den höher schmelzenden/erweichenden Kern obige Schmelz/Erweichungs- und Schrumpfeinschränkungen gelten.

Auch unverstreckte oder teilverstreckte Fasern, wie z.B. unverstreckte Polyäthylenterephthalat-Fasern sind insbesondere für Bindungszwecke einsetzbar, wenn z.B. die Prägekalanderverfestigungstemperatur der völlig verstreckten Faser so hoch ist, daß der Schrumpf im Kalandernip zu hoch oder unkontrollierbar wird. Auch Verschnitte unverstreckter bzw. teilverstreckter mit vollverstreckten Fasern sind empfehlenswert.

Die beiden gleichen oder ungleichen Faserflore sind hydrophil, d.h. weisen eine Saugkraft für wäßrige Flüssigkeiten auf. Fasern aus an sich hydrophoben Polymeren kann durch Zusatz von Tensiden oder Detergentien in der Polymerschmelzmasse oder aufgetragen von außen auf die Fasern oder der Aquisitions- und Verteilerschicht eine Saugkraft für wäßrige Flüssigkeiten gegeben werden. Bevorzugt werden solche Präparationen, die dem Prdodukt ein dauerhaft netzende Eigenschaften verleihen. Zum Zwecke der dauerhaften Hydrophilie ist aber auch denkbar, hydrophobe Fasern mit regenerierten Cellulosefasern, wie Viscose-Zellwolle, Lyocell, Baumwolle, Flachs, Wolle und dergleichen, zu verschneiden. Die Erfindung schließt auch eine Aquisitions - und Verteilerschicht ein, bei denen ein Hydrophilie-Gradient von der Schicht 31 mit einer niedrigeren in Richtung der Schicht 32 mit einer höheren Hydrophilie herrscht.

Dies kann beispielsweise dadurch erreicht werden, daß der prozentuale Anteil der hydrophilen Fasern in Schicht 32 höher gewählt worden ist als in Schicht 31.

Das Verfahren läuft wie folgt ab: Zwischen zwei Faserfloren mit gleichem oder ungleichem Gewicht, gleicher oder ungleicher Orientierung und Zusammensetzung wird ein zur Schrumpfung fähiges Plastiknetz, Gelege oder anderes schrumpffähiges, mit Durchbrechungen versehenes Flächengebilde gelegt und einem Kalander zur Verfestigung des dreischichtigen Schichtstoffes zugeführt.

Die Winkel der sich kreuzenden geradlinigen Fäden des Netzes kann >0 bis 90° sein, wobei bei Winkeln ungleich 90° die sich kreuzenden Fäden 2 x Winkel < 90° und 2 Winkel mit 180° minus des Betrages des Winkels < 90° ergeben, nachdem bekanntlich die Summe der 4 Winkel an den Kreuzungspunkten insgesamt immer 360° ergeben müssen.

In einer bevorzugten Ausführungform beträgt der Winkel der sich kreuzenden Fäden des Netzes 4 x 90°. Die in einer Richtung orientierten geradlinigen Fäden sind jeweils parallel zueinander angeordnet und die Winkel der sich kreuzenden Fäden sind über die gesamte Netzfläche verteilt gleich. Die Abstände der parallel ausgerichteten Fadenpaare können gleich oder unterschiedlich sein. Vorzugsweise werden solche mit gleichen parallelen Abständen eingesetzt.

Die sich kreuzenden Fäden können außer geradlinig auch eine geschwungene oder sonstige Form einnehmen. Die geometrische Form kann sich über die Fadenlänge ausgedehnt in regelmäßigen oder unregelmäßigen Abständen wiederholen. Die geschwungenen oder sonstigen geometrischen Linien der Fäden kann so stark ausgebildet sein, daß sie sich mit denen der benachbarten ebenso ausgerichteten Fäden überschneiden oder kreuzen.

Jede beliebige Anordnung von schrumpffähigem Polymermaterial in Faden und anderer Form und Abwechslung mit Perforationen kann für die Erfindung eingesetzt werden.

Das Netz kann in einer Vorzugsrichtung dickere Fäden aufweisen oder aus Fäden bestehen, die in einer Richtung eine höhere Schrumpfkraft als in der anderen aufweisen. Aus Kostengründen werden vorzugsweise Netze aus Polyolefinen und ganz bevorzugt aus Polypropylen verwendet.

Die drei zunächst miteinander nicht verbunden Schichten werden einem Kalander zugeführt, der aus einer beheizten, gravierten und einer glatten Stahlwalze besteht und dort durch Passage desselben zu einem Komposit vernietet. Unmittelbar nach dem Verlassen des Kalanderwalzen-Nips erfährt die Ware einen Flächenschrumpf, d.h. einen Schrumpf sowohl in Längs- als auch in Querrichtung. Mit dem Fachmann bekannten technischen Mitteln, wie z.B. Temperatursteuerung bzw. Temperaturabführung und/oder Breitstreckhaltung mittels eines Spannrahmens wird ein verstärkter Querschrumpf im Randbereich der Ware verhindert. Es entsteht eine Ware mit Erhebungen, deren Form und Höhe von der Konstruktion des Netzes und den Schrumpfbedingungen abhängt.

Es überraschte, daß die extrem niedrige Aufenthaltsdauer in dem Kalandernip zur Schrumpfung bereits ausreicht.

### Beispiel

Ein biaxial gerecktes Plastiknetz auf Basis Polypropylen mit einem Gewicht von 11 g/m² und einer Mesh Size von 5,0 x 5,0 mm wird zwischen zwei quergelegte lose Stapelfaserflore mit einem Gewicht von jeweils 20 g/m² gelegt. Die beiden Stapelfaserflore sind aus 100% gekräuselten Polyäthylenterephthalat-Fasern mit einem Titer von 6,7 dtex und einer Schnittlänge von 60 mm aufgebaut. Die drei Schichten werden einer Punktverschweißung durch Kalandrierung zwischen einer glatten und einer gravierten Stahlwalze zugeführt. Die Verschweißfläche der gravierten Walze beträgt 9,6 % und die Gravurtiefe 0,73 mm. Kalandriert wurde bei einer Temp. von 198°C auf beiden Walzen und einem Liniendruck von 30 kp/cm bei einer Geschwindigkeit von 10 m/min. Die Breite der Ware vor dem Schrumpf betrug 110 cm. Unmittelbar nach dem Verlassen des Kalandernips bildeten sich in der Ware wellenförmige Erhebungen, die sich senkrecht zur Maschinenrichtung beinahe über die gesamte Breite der Ware ausbreiten. Lediglich in einem Randbereich von jeweils ca. 7 cm Breite auf beiden Seiten bildeten sich durch Längs- und Querschrumpf bergkuppenförmige Erhebungen nach oben und unten und eine überdimensionale Gewichtserhöhung. Dieser Randbereich wird wegen fehlender Gewichtskonstanz und der andersartigen Struktur abgeschnitten und nicht als Aufnahme- und Verteilerschicht verwendet. Die Warenbreite nach der Kalanderpassage reduzierte sich von 110 auf 92 cm. Durch Abtrennung des nicht verwertbaren Randes 2 x ca. 7 cm reduziert sich die Nutzbreite auf 78 cm. In diesem Bereich stellte sich ein Flächenschrumpf in Längsrichtung von 55,1% nach der Kalanderpassage ein. Das Ausgangsgewicht von 51,0 g/m^{2,} nämlich 2 x 20 g/m² für die Stapelfaserflore und 11 g/m² für das Plastiknetz, erhöhte sich, so dass das Komposit-Gewicht 114 g/m² betrug.

Die Prüfungen wurden innerhalb der Nutzbreite von 78 cm durchgeführt. Bestimmt wurden das Schichtstoffgewicht in g/m², die Dicke in mm bei einem Auflagedruck von 8 g/cm². Für die Berechnung des Kompressionswiderstandes KW wurde zusätzlich die Dicke bei 64 g/cm² ermittelt.

Man erhält der Wert für KW in %, indem man die Dicke bei 8g/cm² durch die Dicke bei 64 g/cm² teilt und mit 100 multipliziert.

Zur Bestimmung des Wiederholungsvermögens W wird wie folgt vorgegangen:
Die Dicke D1 wird bei einer Belastung von 8 g/cm² nach 30 Sekunden gemessen. Danach wird am gleichen Probekörper und genau dergleichen Prüfstelle die Belastung durch ein entsprechendes Zusatzgewicht auf 64 g/cm² erhöht und wieder nach 30 Sekunden Belastungszeit die Dicke ermittelt. Anschließend wird dieses Zusatzgewicht wieder entfernt, so daß auf den gleichen Probekörper mit genau dergleichen Prüfstelle wieder ein Druck von 8 g/cm² lastet. Nach einer Belastungszeit von wieder 30 Sekunden wird die Dicke in mm erneut gemessen (D2). Das Wiederholungsvermögen W in % errechnet sich durch Division von D2 durch D1 und Multiplikation mit 100.

Unter Kriechbeständigkeit KB verstehen wir die verbleibende Dicke in % nach einer Dauerbelastung von 35,4 g/cm² der Ware gelagert über eine Dauer von 24 Stunden im Trockenschrank bei 60°C im Vergleich zur Dicke der Probe gemessen bei einer Belastung von 8 g/cm² nach 30 Sekunden.

Die Bestimmung des KB im einzelnen erfolgt wie in den nächsten Zeilen beschrieben: 5 Prüflinge vom Format 90 x 90 mm werden ausgeschnitten. Es wird daran die Dicke bei 8 g/cm² Belastung nach 30 Sekunden gemessen. Anschließend werden die Proben mit einem runder Zylinder von 1 kg-Gewicht mit einem Durchmesser von 60 mm; (entspricht 3500 Pa = 35,4 g/cm²) belastet und mit dieser Belastung über einen Zeitraum von 24 Stunden im Trockenschrank bei 60°C gelagert. Nach der Entfernung des Gewichts werden die Proben aus dem Trockenschrank genommen und verbleiben 2 Minuten lang Zeit im unbelasteten Zustand. Anschließend wird die Dicke der Proben bei 8 g/cm² Belastung gemessen. Die Dicke nach der 24-stündigen Belastung bei 60°C geteilt durch die Ausgangsdicke, jeweils gemessen bei 8 g/cm², multipliziert mit 100 ergibt KW in %.

## Patentansprüche

1. Schichtstoff, aufweisend eine erste Deckschicht, ein Durchbrechungen aufweisendes Flächengebilde als Mittelschicht und eine zweite Deckschicht, wobei das Flächengebilde in geschrumpftem Zustand vorliegt.

2. Schichtstoff nach Anspruch 1, **dadurch gekennzeichnet, daß** das Flächengebilde in einer Richtung eine höhere Schrumpfkraft als in der anderen Richtung aufweist.

3. Schichtstoff nach Anspruch 1 oder 2, **dadurch gekennzeichnet, daß** die erste und die zweite Deckschicht aus Fasern und/oder Filamenten bestehen, deren Schmelz- oder Erweichungspunkt mindestens 30°C über dem Schmelz- oder Erweichungspunkt des Flächengebildes liegt.

4. Schichtstoff nach einem der Ansprüche 1 bis 3, **dadurch gekennzeichnet, daß** mindestens eine Deckschicht aus gekräuselten Stapelflaserfloren oder Endlosfilamenten besteht.

5. Schichtstoff nach einem der Ansprüche 1 bis 4, **dadurch gekennzeichnet, daß** mindestens eine Deckschicht aus einem vorgebundenen Flächengebilde, beispielsweise Papier oder Vliesstoff, besteht.

6. Schichtstoff nach einem der Ansprüche 1 bis 5, **dadurch gekennzeichnet, daß** in mindestens einer Deckschicht zumindest anteilig thermoplastisch verformbares und verschweißbares Material vorhanden ist.

7. Schichtstoff nach einem der Ansprüche 1 bis 6, **dadurch gekennzeichnet, daß** das Flächengebilde in der Mittelschicht ein Gelege ist, dessen Fäden sich an den sich kreuzenden Stellen mit Hilfe eines zusätzlichen Haft- oder Bindemittels aneinander gebunden sind.

8. Schichtstoff nach einem der Ansprüche 1 bis 6, **dadurch gekennzeichnet, daß** das Flächengebilde aus einem Netz aus Polymermasse gebildet ist, deren sich kreuzende Fäden durch die eigene Polymermasse miteinander verschweißt oder aneinander gebunden sind.

9. Schichtstoff nach einem der Ansprüche 1 bis 8, **dadurch gekennzeichnet, daß** die sich kreuzenden geradlinigen Fäden des Flächengebildes in einem Winkel von 90° zueinander angeordnet sind.

10. Schichtstoff nach einem der Ansprüche 2 bis 9, **dadurch gekennzeichnet, daß** das Flächengebilde in einer Vorzugsrichtung aus schrumpffähigem und in einer bezogen auf die Vorzugsrichtung verlaufenden Querrichtung aus unter Prozeß- und Anwendungsbedingungen nicht schrumpffähigem Material gebildet ist.

11. Schichtstoff nach einem der Ansprüche 2 bis 10, **dadurch gekennzeichnet, daß** das Flächengebilde sehr grobe Monofilamente in Längs - und sehr feine Monofilamente in Querrichtung aus dem selben Polymer, beispielsweise Polypropylen, gebildet ist.

12. Schichtstoff nach Anspruch 11, **dadurch gekennzeichnet, daß** an den Längsfäden die Schmelzmasse der aufgeschmolzenen Querfäden angesammelt ist.

13. Schichtstoff, aufweisend eine erste Deckschicht, eine Mittelschicht aus sich nicht überkreuzenden Fäden und eine zweite Deckschicht, wobei die sich nicht überkreuzenden Fäden in geschrumpften Zustand vorliegen.

14. Schichtstoff nach Anspruch 13, umfassend drei Schichten, wobei die mittlere Schicht aus längs ausgerichteten parallelen Monofilamentfäden aus einem ersten Thermoplasten besteht und wobei ober- und/oder unterhalb der Fäden des ersten Thermoplasten Monofilamentfäden aus einem zweiten Thermpolasten abgelegt sind, wobei die zweiten Monofilamentfäden derart angeordnet sind, daß mindestens zwei parallel und linear ausgerichtete Fäden des ersten Thermoplast gekreuzt und verbunden sind.

15. Schichtstoff nach Anspruch 14, **dadurch gekennzeichnet, daß** die ersten Monofilamentfäden einen um mindestens 30° höheren Schmelz- oder Erweichungspunkt aufweisen.

16. Schichtstoff nach einem der Ansprüche 14 oder 15, **dadurch gekennzeichnet, daß** die zweiten Monofilamentfäden sinusförmig über die längs ausgerichteten ersten Monofilamentfäden abgelegt sind.

17. Schichtstoff nach einem der Ansprüche 14 bis 16, **dadurch gekennzeichnet, daß** die zweiten Monofilamente Bikomponenten-Struktur aufweisen, wobei zumindest eine Polymerkomponente einen um mindesten 30° gegenüber den ersten Monofilamentfäden aus einem ersten Thermoplast höheren Schmelz- bzw. Erweichungspunkt aufweist.

18. Schichtstoff nach einem der Ansprüche 1 bis 17, **dadurch gekennzeichnet, daß** der Schrumpf in Längsrichtung 20 bis 75 %, vorzugsweise 30 bis 60 % bezogen auf die Länge der ungeschrumpften Ausgangsbasis beträgt.

19. Schichtstoff nach einem der Ansprüche 1 bis 18, mit einem Flächengewicht von 50 bis 200 g/m², vorzugsweise 70 bis 150 g/m², wobei die zweite Schicht 5 bis 50 %, vorzugsweise 10 bis 30 % des Gesamtgewichts ausmacht.

20. Schichtstoff nach einem der Ansprüche 1 bis 19, **dadurch gekennzeichnet, daß** der Schmelz- oder Erweichungspunkt der Fasern der Deckschicht mindestens 20 bis 30°C über dem der Mittelschicht bzw. dem niedrigst schmelzenden Teil der Mittelschicht liegt und keinen oder im Vergleich zur Mittelschicht nur einen unbedeutenden Flächenschrumpf aufweist.

21. Schichtstoff nach einem der Ansprüche 1 bis 20, **dadurch gekennzeichnet, daß** die erste und/oder die zweite Schicht aus hydrophilen Faserfloren gebildet sind, wobei vorzugsweise ein Hydrophilie-Gradient zwischen der ersten und der zweiten Deckschicht vorhanden ist.

22. Verwendung des Schichtstoffs nach einem der vorhergehenden Ansprüche 1 bis 21 für eine Fluidaufnahme- und/oder Verteilerschicht für absorbierende Hygiene-Artikel.

23. Verfahren zur Herstellung eines mindestens drei Schichten aufweisenden Schichtstoffs, **dadurch gekennzeichnet, daß** zwischen zwei Deckschichten eine Mittelschicht in Form eines Flächengebildes oder in Form von Fäden eingebracht wird, wobei in einem zweiten Schritt das Flächengebilde oder die Längsfäden bei einer Temperatur oberhalb der Schmelz- oder Erweichungstemperatur des Materials der Mittelschicht im Walzenspalt geschrumpft werden.

24. Verfahren nach Anspruch 23, **dadurch gekennzeichnet, daß** mindestens eine Walze mit einem unterbrochenen Muster, beispielsweise Punkten, graviert ist.

25. Verfahren nach Anspruch 23 oder 24, **dadurch gekennzeichnet, daß** die drei Schichten einem Kalander mit mindestens einer beheizten, gravierten Stahlwalze zugeführt werden und dort zu einem Komposit verbunden werden, wobei die Verbindung insbesondere als Vernieten zu bezeichnen ist.

26. Verfahren nach einem der Ansprüche 23 bis 25, **dadurch gekennzeichnet, daß** im Kalanderspalt die Querfäden durchgeschmolzen, durchgetrennt bzw. stark geschädigt werden.

27. Verfahren nach einem der Ansprüche 23 bis 26, **dadurch gekennzeichnet, daß** die Fäden bogenförmig auf der ersten Deckschicht abgelegt werden.

28. Verfahren nach Anspruch 27, **dadurch gekennzeichnet, daß** die Fäden untereinander kreuzungsfrei abgelegt werden.
